# EUROPÄISCHE PATENTANMELDUNG

(11) **EP 4 809 936 A1**
(43) Veröffentlichungstag der Anmeldung: **23.09.2026**
(21) Anmeldenummer: 26157434.7
(22) Anmeldetag: 10.02.2026
(51) Int. Cl.: A61B 17/02

(54) **MEDIZINISCHES INSTRUMENT**

(30) Priorität: 19.03.2025 DE 102025110599
(71) Anmelder: Tontarra Medizintechnik GmbH, 78573 Wurmlingen (DE)
(72) Erfinder: TONTARRA, Thomas, 78573 Wurmlingen (DE); NEUBRAND, Florian, 78194 Immendingen (DE); TRUCKENBROD, Stefan, 78576 Emmingen-Liptingen (DE)
(74) Vertreter: Mammel und Maser Patentanwälte PartG mbB

(57) **Zusammenfassung**

Die Erfindung betrifft ein medizinisches Instrument (11) mit zwei Branchen (14, 15), die jeweils einen Griffabschnitt (18, 19) und Funktionsabschnitte (21, 22) aufweisen, zwischen denen ein Schlussabschnitt (23, 24) vorgesehen ist, mit einem Schluss (16), durch welchen die Branchen (14, 15) schwenkbar um eine gemeinsame Drehachse (17) verbunden sind, wobei die Schlussabschnitte (23, 24) mit einem Verbindungselement (26) schwenkbar in der Drehachse (17) gelagert sind, mit einer Verriegelungseinrichtung (28), welche zwei voneinander lösbare Verriegelungselemente (31, 32) aufweist, wobei eines der Verriegelungselemente (31) mit einem Verriegelungshebel (29) betätigbar ist, wobei die Verriegelungselemente (31, 32) an oder in dem Schluss (16) angeordnet sind.

## Beschreibung

Die Erfindung betrifft ein medizinisches Instrument mit zwei Branchen, die durch einen Schluss um eine gemeinsame Drehachse schwenkbar verbunden sind.

Aus der GB 1 260 101 A ist ein Retraktor bekannt, welcher zwei Branchen umfasst, die jeweils einen Griffabschnitt und einen Funktionsabschnitt aufweisen, zwischen denen ein Schlussabschnitt vorgesehen ist. Die beiden Schlussabschnitte der Branchen bilden einen Schluss, durch welchen die Branchen schwenkbar und in einer gemeinsamen Drehachse miteinander verbunden sind. Zwischen dem Schluss und den Grifföffnungen der Griffabschnitte ist eine Verriegelungseinrichtung vorgesehen. An dem einen Griffabschnitt ist ein kreisbogenförmig ausgebildetes Verriegelungselement befestigt, welches sich durch einen Schlitz in dem gegenüberliegenden Griffabschnitt erstreckt. An dem gegenüberliegenden Griffabschnitt ist ein Verriegelungshebel schwenkbar gelagert, durch welchen das zweite Verriegelungselement lösbar zum ersten Verriegelungselement ist.

Solche medizinischen Instrumente weisen den Nachteil auf, dass die Griffabschnitte in einer Arbeitsposition nicht vollständig aneinanderliegend positionierbar sind, da auf einer Innenseite des einen Griffabschnittes ein Federelement zur Sicherung des Verriegelungshebels in einer Verriegelungsposition angeordnet ist. Darüber hinaus sind die Verriegelungselemente nahe dem Griffabschnitt offen ausgebildet und frei zugänglich, wodurch es aufgrund der Verzahnung der Verriegelungselemente zu Verletzungen von OP-Handschuhen kommen kann.

Aus der DE 11 2012 002 181 T5 ist ein weiterer Retraktor bekannt. Der Aufbau und die Anordnung der Verriegelungseinrichtung an den Griffabschnitten der Branchen ist analog zu dem vorstehend zitierten Retraktor ausgebildet.

Der Erfindung liegt die Aufgabe zugrunde, ein medizinisches Instrument zu schaffen, welches eine verbesserte Handhabung ermöglicht.

Diese Aufgabe wird durch ein medizinisches Instrument gelöst, welches zwei Branchen umfasst, die jeweils einen Griffabschnitt und einen Funktionsabschnitt aufweisen, zwischen denen ein Schlussabschnitt vorgesehen ist, wobei die Branchen schwenkbar um eine gemeinsame Drehachse durch einen Schluss miteinander verbunden sind, wobei die Schlussabschnitte mit einem Verbindungselement schwenkbar um die Drehachse zur Bildung des Schlusses gelagert sind. Des Weiteren ist eine Verriegelungseinrichtung vorgesehen, welche zwei voneinander lösbare Verriegelungselemente aufweist, wobei eines der Verriegelungselemente mit einem Verriegelungshebel betätigbar ist. Die Verriegelungselemente sind an oder in dem Schluss angeordnet. Dies ermöglicht, dass die Verriegelungselemente nicht offen oder frei an einem Griffabschnitt ausgebildet sind, sondern an oder in dem Schluss geschützt vorgesehen sind. Dies ermöglicht, dass die Verriegelungselemente nicht offen und frei zugänglich sind, sodass die Verletzung von OP-Handschuhen nicht mehr gegeben ist.

Bevorzugt ist vorgesehen, dass die Verriegelungselemente innerhalb des Schlusses integriert sind. Dadurch sind diese Verriegelungselemente von außen nicht zugänglich und vorzugsweise auch nicht oder nur bedingt sichtbar.

Die Verriegelungselemente umfassen bevorzugt aufeinander zuweisende Rastelemente, die ineinandergreifen. Dadurch kann eine stufenweise Öffnungs- oder Schließstellung der Branchen einstellbar sein.

Insbesondere ist vorgesehen, dass die Rastelemente der Verriegelungselemente derart ausgebildet sind, dass eine Schwenkbewegung der Branchen in eine Richtung, insbesondere in eine Schließrichtung der Funktionsabschnitte der Branchen, gesperrt ist und eine Schwenkbewegung der Branchen in eine andere Richtung, insbesondere in eine Aufspreizrichtung der Funktionsabschnitte der Branchen, freigegeben wird. Diese Funktionsweise der Verriegelungselemente ist insbesondere bei sogenannten Retraktoren vorgesehen.

Gemäß einer bevorzugten Ausführungsform ist vorgesehen, dass das eine Verriegelungselement fest an dem einen Schlussabschnitt ausgebildet ist und das andere an dem Verriegelungshebel angeordnete Verriegelungselement lösbar dazu ausgerichtet ist. Dadurch kann ein konstruktiv kompakter Aufbau der Verriegelungseinrichtung geschaffen werden.

Der Verriegelungshebel ist bevorzugt an dem Griffabschnitt schwenkbar gelagert, welcher dem Griffabschnitt mit dem in dem Anschlussabschnitt fest angeordneten Verriegelungselement gegenüberliegt. Diese Anordnung weist den Vorteil auf, dass die beiden Griffabschnitte frei ausgebildet sind und nicht durch ein Ineinandergreifen von zumindest einem Rastelement konstruktiv aufwendig ausgebildet ist.

Insbesondere ist vorgesehen, dass der Verriegelungshebel an einer Außenseite des Griffabschnittes der einen Branche angeordnet ist, sodass die Griffabschnitte der beiden Branchen in einer Arbeitsposition unmittelbar aneinanderliegend positionierbar sind. Dadurch kann eine vergrößerte Öffnungsweite der Funktionsabschnitte einstellbar sein.

Der Verriegelungshebel ist bevorzugt um eine Schwenkachse parallel zur Drehachse des Schlusses schwenkbar gelagert. Insbesondere ist dabei vorgesehen, dass das an dem Verriegelungshebel angeordnete Verriegelungselement und vorzugsweise zumindest teilweise ein Hebelarm des Verriegelungshebels innerhalb des Schlussabschnittes angeordnet ist. Somit ist durch eine Aussparung in dem Griffabschnitt die Anordnung und Führung eines Hebelarmes mit dem Verriegelungselement, als auch die Gelenkverbindung von dem Verriegelungshebel zu dem Griffabschnitt möglich.

Bevorzugt ist vorgesehen, dass der Verriegelungshebel selbsthaltend durch ein Kraftspeicherelement in einer Verriegelungsposition angeordnet ist, sodass die Verriegelungselemente ineinandergreifen oder aneinandergreifen. Dadurch kann insbesondere eine einmal eingestellte und aufgespreizte Position der Funktionselemente aufrechterhalten werden.

Das auf den Verriegelungshebel wirkende Kraftspeicherelement ist bevorzugt als ein Federelement ausgebildet. Dieses Federelement ist aus einer für medizinische Instrumente zugelassenen Legierung ausgebildet, sodass auch eine einfache Reinigung und Sterilisierung ermöglicht ist.

Vorteilhafterweise ist vorgesehen, dass in dem Schlussabschnitt ein federgelagertes Druckstück vorgesehen ist, welches an dem Verriegelungselement angreift oder anliegt. Das Druckstück greift an der dem Rastelement gegenüberliegenden Seite des Verriegelungselementes an. Dadurch kann in einfacher Weise die selbsthaltende Verriegelung ermöglicht sein.

Insbesondere ist vorgesehen, dass das federgelagerte Druckstück entlang einer Achse verschiebbar geführt ist, welche senkrecht zur Drehachse des Schlusses ausgerichtet ist und insbesondere diese Drehachse schneidet. Dadurch können gleichbleibende Kraftverhältnisse in Verriegelungsrichtung unabhängig einer Winkelposition der zwei Funktionsabschnitte der Branchen zueinander gegeben sein.

Des Weiteren ist bevorzugt vorgesehen, dass sich der Verriegelungshebel bis zu einer Grifföffnung des Griffabschnittes erstreckt. Dadurch ist eine einfache und ergonomische Handhabung des medizinischen Instrumentes ermöglicht.

Vorteilhafterweise umfassen die Funktionsabschnitte jeweils eine Schnittstelle zur lösbaren Anordnung von Funktionselementen. Dies ermöglicht, dass dieses medizinische Instrument für verschiedene Einsatzfälle umrüstbar ist. Beispielsweise handelt es sich um sogenannte Gelpi-, Weitlaner-, Plester-, Adson- oder Beckmann-Funktionselemente oder dergleichen.

Der Schluss, welcher die zwei Branchen des medizinischen Instrumentes um eine gemeinsame Drehachse schwenkbar miteinander verbindet, kann als ein sogenannter aufgelegter Schluss oder Rollenschluss oder als ein Durchsteck- oder Kastenschluss ausgebildet sein.

Die Erfindung sowie weitere vorteilhafte Ausführungsformen und Weiterbildungen derselben werden im Folgenden anhand der in den Zeichnungen dargestellten Beispiele näher beschrieben und erläutert. Die der Beschreibung und den Zeichnungen zu entnehmenden Merkmale können einzeln für sich oder zu mehreren in beliebiger Kombination erfindungsgemäß angewandt werden. Es zeigen:
- Figur 1: eine perspektivische Ansicht auf das medizinische Instrument in einer ersten Arbeitsposition,
- Figur 2: eine Seitenansicht auf das medizinische Instrument gemäß Figur 1 in einer weiteren Arbeitsposition,
- Figur 3: eine schematische Seitenansicht des medizinischen Instrumentes gemäß Figur 1 in einer teilweisen Schnittansicht,
- Figur 4: eine schematische Seitenansicht des medizinischen Instrumentes gemäß Figur 2 in einer teilweisen Schnittansicht,
- Figur 5: eine schematisch vergrößerte Detailansicht einer Verriegelungseinrichtung des medizinischen Instrumentes gemäß Figur 3 in einer Entriegelungsposition,
- Figur 6: eine schematisch vergrößerte Detailansicht der Verriegelungsvorrichtung des medizinischen Instrumentes gemäß Figur 4 in einer Verriegelungsposition,
- Figur 7: eine schematisch vergrößerte Seitenansicht eines Durchsteckschlusses gemäß Figur 1,
- Figur 8: eine schematisch vergrößerte Stirnansicht des Durchsteckschlusses gemäß Figur 7,
- Figur 9: eine schematisch vergrößerte Seitenansicht eines aufgelegten Schlusses,
- Figur 10: eine schematische Schnittansicht des aufgelegten Schlusses gemäß Figur 9,
- Figur 11: eine schematische Schnittansicht des aufgelegten Schlusses gemäß Figur 9 in einer Entriegelungsposition,
- Figur 12: eine schematische Schnittansicht des aufgelegten Schlusses gemäß Figur 9 in einer weiteren Arbeitsposition,
- Figur 13: eine schematische Seitenansicht des aufgelegten Schlusses gemäß Figur 12,
- Figur 14: eine perspektivische Ansicht einer alternativen Ausführungsform des aufgelegten Schlusses zu Figur 9,
- Figur 15: eine schematische Schnittansicht des Schlusses gemäß Figur 14 in einer Verriegelungsposition,
- Figur 16: eine perspektivische Ansicht einer alternativen Ausführungsform des aufgelegten Schlusses gemäß Figur 14 in einer Entriegelungsposition, und
- Figur 17: eine schematische Schnittansicht des Schlusses gemäß Figur 16.

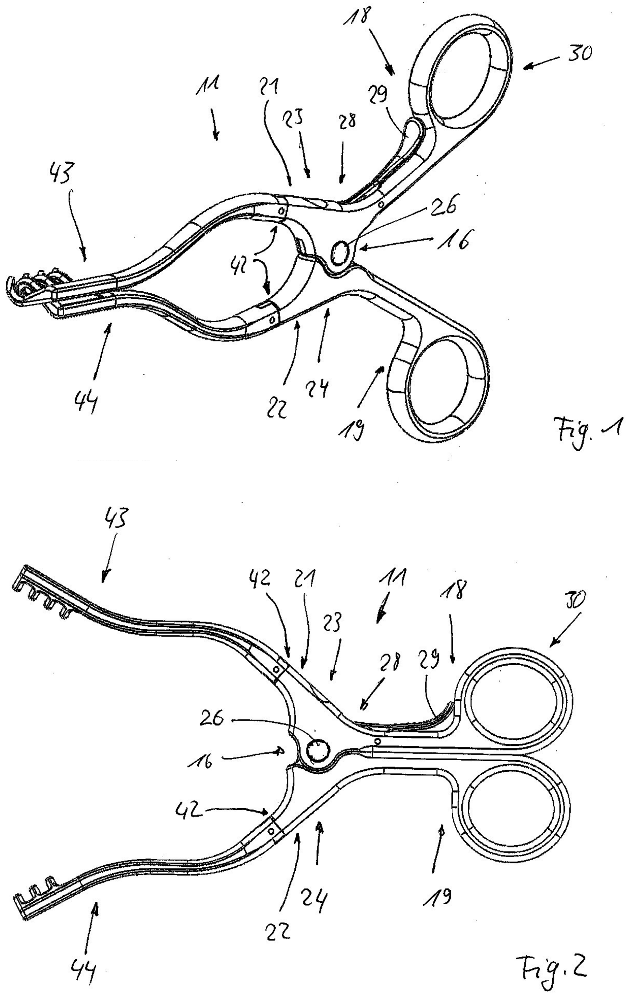

In Figur 1 ist ein medizinisches Instrument 11 in einer perspektivischen Ansicht in einer Arbeitsposition, insbesondere einer geschlossenen Arbeitsposition dargestellt. Die Figur 2 zeigt eine schematische Seitenansicht des medizinischen Instrumentes 11 gemäß Figur 1 in einer weiteren Arbeitsposition, insbesondere einer geöffneten Arbeitsposition.

Das medizinische Instrument 11 umfasst zwei Branchen 14, 15, die durch einen Schluss 16 schwenkbar um eine gemeinsame Drehachse 17 miteinander verbunden sind. Jede Branche 14, 15 besteht aus einem Griffabschnitt 18, 19 sowie einem Funktionsabschnitt 21, 22 und dazwischenliegend einen Schlussabschnitt 23, 24. Die Schlussabschnitte 23, 24 sind durch ein Verbindungselement 26 in der Drehachse 17 des Schlusses 16 schwenkbar miteinander verbunden. Bei diesem Verbindungselement 26 kann es sich um eine Schraube, einen Stift oder dergleichen handeln.

Am jeweiligen proximalen Ende des Funktionsabschnitts 21, 22 ist bevorzugt eine Schnittstelle 42 vorgesehen, welche zur Anbindung von einem Funktionselement 43, 44 dient. Diese Funktionselemente 43, 44 können in einer festen, also unlösbaren Anordnung zum Funktionsabschnitt 21, 22 angeordnet sein. Alternativ besteht auch die Möglichkeit, dass die Schnittstelle 43 mit dem jeweiligen Funktionselement 43, 44 eine lösbare Anordnung bildet, sodass auch ein einfacher Austausch von Funktionselementen 43, 44 an den Branchen 14, 15 möglich ist. Des Weiteren kann vorgesehen sein, dass die Funktionsabschnitte 21, 22 und die Funktionselemente 43, 44 auch jeweils einteilig ausgebildet sind, sodass die Branche 14, 15 jeweils aus dem Griffabschnitt 18, 19, dem Schlussabschnitt 23, 24 sowie den Funktionselement 43, 44 besteht.

Dieses medizinische Instrument 11 umfasst des Weiteren eine Verriegelungseinrichtung 28, von welcher in den Ansichten in Figur 1 und 2 nur ein Griffbereich des Verriegelungshebels 29 sichtbar ist. Dieser Verriegelungshebel 29 erstreckt sich in Richtung einer Grifföffnung 30 des Griffabschnittes 18, wodurch eine ergonomische Handhabung ermöglicht ist. Die Verriegelungseinrichtung 28 ist nachfolgend in den Figuren näher beschrieben.

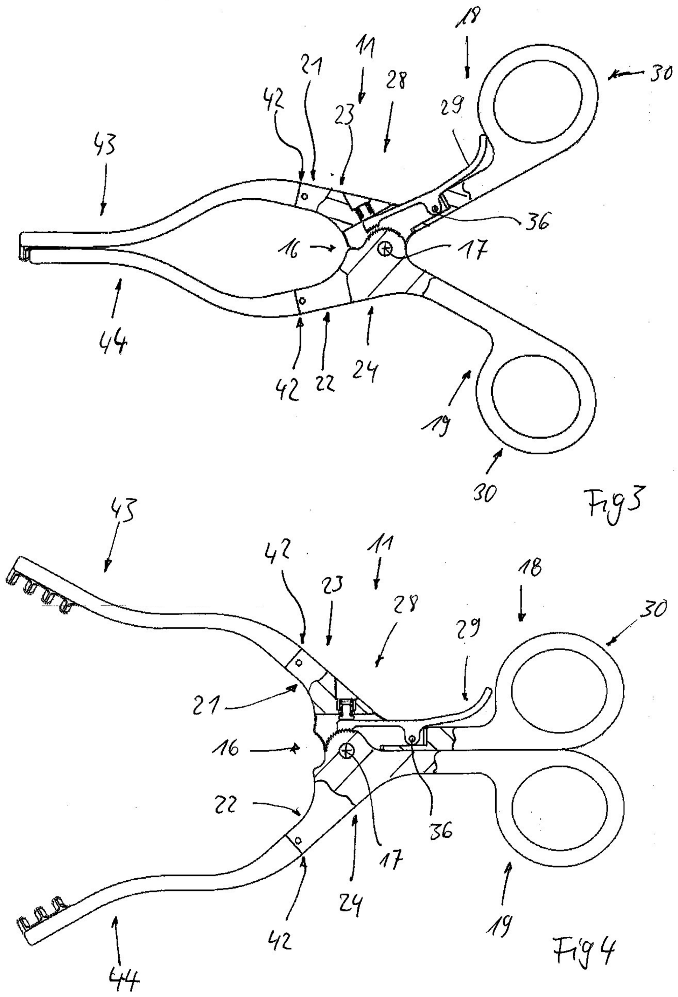

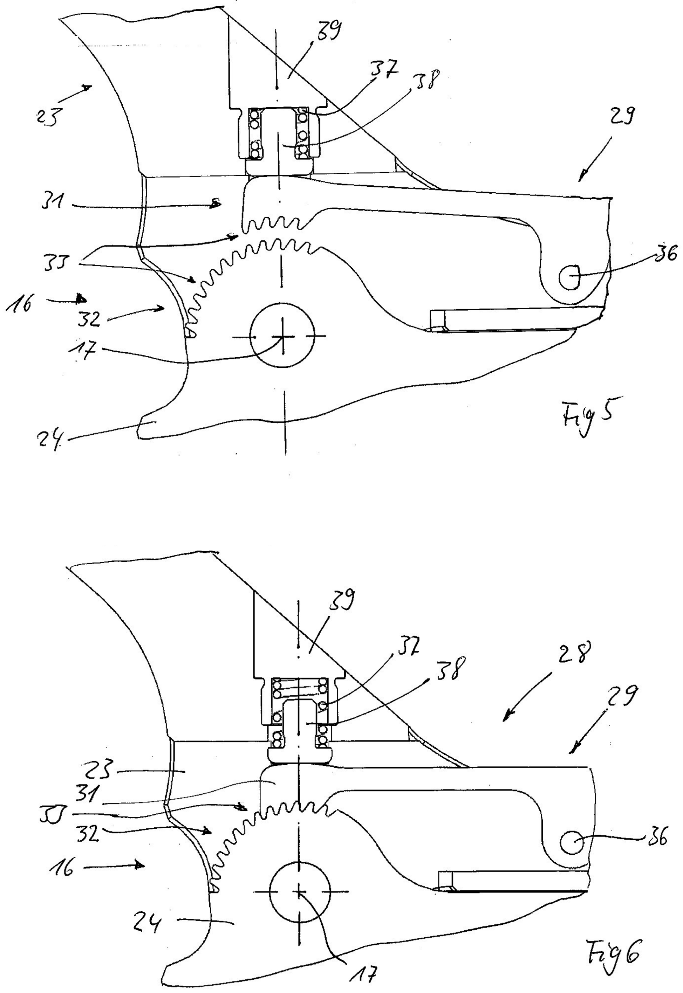

In Figur 3 ist eine schematische Seitenansicht im Teilschnitt des medizinischen Instrumentes 11 gemäß Figur 1 dargestellt. Die Verriegelungseinrichtung 28 ist geöffnet bzw. entsperrt. In Figur 4 ist eine schematische Ansicht des medizinischen Instrumentes 11 gemäß Figur 2 in einem Teilschnitt dargestellt. Bei dieser Ausführungsform ist die Verriegelungseinrichtung 28 geschlossen. Die Figur 5 zeigt schematisch vergrößert die Verriegelungseinrichtung 28 gemäß Figur 3. Die Figur 6 zeigt schematisch vergrößert die Verriegelungseinrichtung 28 gemäß Figur 4.

Die Verriegelungseinrichtung 28 umfasst zwei Verriegelungselemente 31, 32 mit einander zuweisenden Rastelementen 33. Bei solchen Rastelementen 33 kann es sich beispielsweise um Zähne handeln, die verrastend ineinandergreifen. Das eine Verriegelungselement 32 ist fest an dem Schlussabschnitt 24 der Branche 15 vorgesehen. Das Verriegelungselement 32 ist auf einem Kreisabschnitt fest angeordnet, dessen Mittelpunkt in der Drehachse 17 liegt. Der Durchmesser des Kreisabschnittes und das daran angeordnete Verriegelungselement 32 sind im Abstand zur Drehachse 17 derart ausgebildet, dass diese innerhalb des benachbarten oder daran angrenzenden Schlussabschnittes 23 liegen.

Dieser Schlussabschnitt 23 der Branche 14 weist im Bereich des Schlusses 16 eine Aussparung, insbesondere U-förmige Aussparung, auf, sodass das weitere Verriegelungselement 31 in dieser Aussparung positionierbar und bewegbar ist. Dieses Verriegelungselement 31 ist an einem Ende des Verriegelungshebels 29 angeordnet. An dem gegenüberliegenden Ende ist der Griffbereich des Verriegelungshebels 29 nahe der Grifföffnung 31 positioniert. Der Verriegelungshebel 29 ist um eine Schwenkachse 36 schwenkbar gelagert. Diese Schwenkachse 36 ist parallel zur Drehachse 17 ausgerichtet. Der Verriegelungshebel 29 ist um einen Schwenkbereich betätigbar, sodass die Rastelemente 33 von den Verriegelungselementen 31, 32 freikommen. Dabei kann vorgesehen sein, dass der Schwenkbereich durch die Ausformung des Griffbereichs zum Griffabschnitt 18bestimmt ist.

Die Verriegelungseinrichtung 28 ist in einer Verriegelungsposition selbsthaltend ausgebildet (Figur 4). Dies wird dadurch erreicht, dass ein Kraftspeicherelement 37 ein Druckstück 38 beaufschlagt, welches dem Rastelement 33 gegenüberliegend an dem Verriegelungselement 31 angreift. Das Kraftspeicherelement 37 und das Druckstück 38 sind in dem Schlussabschnitt 23 integriert. Diese sind innenliegend angeordnet und bevorzugt von außen nicht sichtbar. Vorzugsweise sind diese durch einen Verschluss 39 in dem Schlussabschnitt 23 gehalten, der in eine Öffnung in dem Schlussabschnitt 23 einsetzbar ist. Bevorzugt ist vorgesehen, dass eine Längsachse des Kraftspeicherelementes 37 und des Druckstückes 38 rechtwinklig zur Drehachse 17 ausgerichtet ist und diese kreuzt. Dadurch wird eine Kraftwirkung erzeugt, die immer axial zur Drehachse 17 wirkt. Dies weist den Vorteil auf, dass unabhängig von einer Arbeitsposition der zwei Branchen 14, 15 zueinander immer gleichgroße Kraftverhältnisse bestehen, um die Verriegelungsposition der Verriegelungseinrichtung 28 durch den Verriegelungshebel 29 zu lösen. Die Abhebebewegung des Verriegelungselementes 31 von dem fest an den Schlussabschnitt 24 angeordeten Verriegelungselement 32 kann durch einen Federweg zwischen dem Druckstück 38 und dem Verschluss 39 begrenzt sein.

Die Rastelemente 33 der Verriegelungselemente 31, 32 sind aufeinander abgestimmt. Die Rastelemente 33 sind bevorzugt sägezahnförmig ausgebildet. Dies bedeutet, dass beispielsweise bei einer Betätigung der Branchen 14, 15 zum Öffnen der Funktionsabschnitte 21, 22 die Verrastung sich selbst löst, sodass die Funktionsabschnitte 21, 22 aufspreizbar sind. In entgegengesetzter Bewegungsrichtung der Funktionsabschnitte 21, 22 erfolgt jedoch ein Sperren, sodass die Funktionsabschnitte 21, 22 nur dann bewegbar sind, sofern die Verriegelungseinrichtung 28 durch den Verriegelungshebel 29 aktiv gelöst wird.

Die in den Figuren 3 bis 6 dargestellte Verriegelungseinrichtung 28 umfasst somit eine rotatorisch angebrachte Sperre. Diese Sperre ist in dem Schluss 16 integriert.

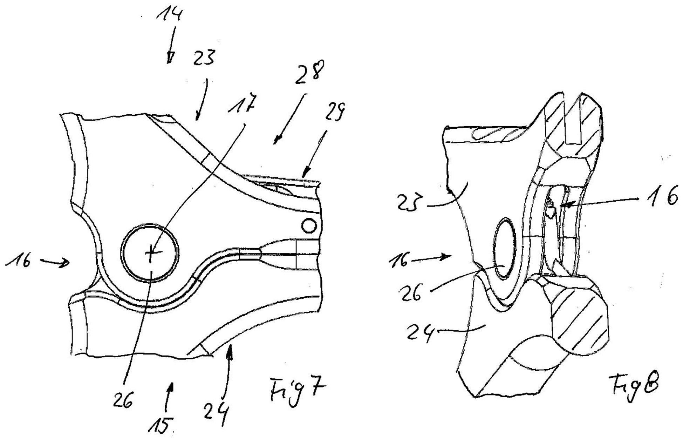

In Figur 7 ist eine schematische Seitenansicht des Schlusses 16 vergrößert dargestellt. Die Figur 8 zeigt eine schematische Ansicht von vorne in dem Schluss 16 gemäß Figur 7. Dieser Schluss 16 wird auch als Rollenschluss bezeichnet. Zur Ausbildung des Schlusses 16 ist vorgesehen, dass an der Branche 15 in der Längsmittelachsenebene der Schlussabschnitt 24 scheiben- oder kreisförmig hervorstehend ausgebildet ist. Der gegenüberliegende Schlussabschnitt 23 der Branche 14 ist durch eine U-förmige Vertiefung ausgebildet, sodass außerhalb des Schlussabschnittes 24 zwei Laschen sich erstrecken und den innenliegenden Schlussabschnitt 24 überdecken. Dieser Schluss 16 kann zur Reinigung gespült werden, da ein freier Durchgang durch den Schlussabschnitt 23, 24 von dem Funktionsabschnitt 21 zu dem Griffabschnitt 18 der Branche 14 gegeben ist.

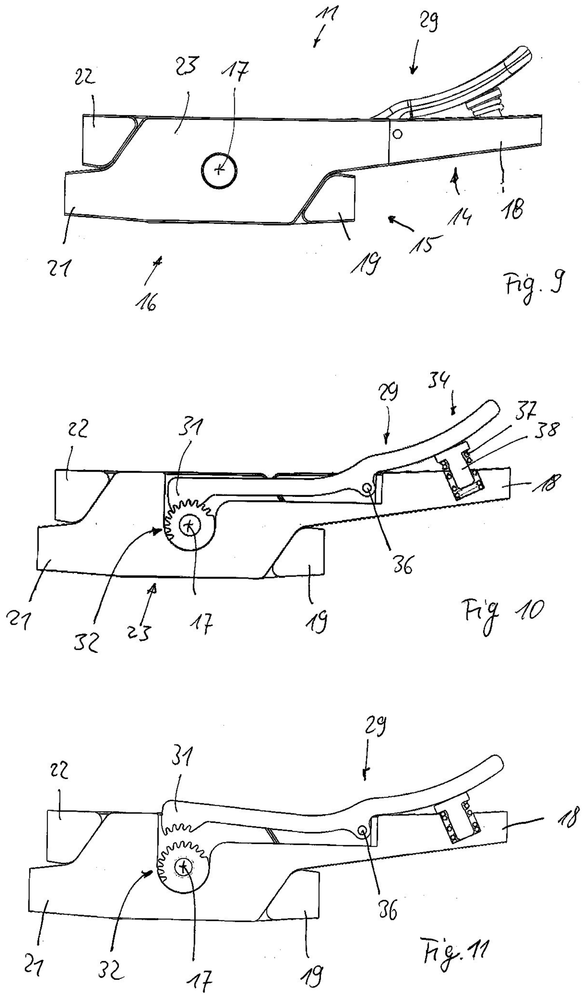

Figur 9 zeigt eine schematische Seitenansicht eines aufgelegten Schlusses 16 in einer Arbeitsposition des medizinischen Instrumentes 11 gemäß Figur 2. Die Figur 10 zeigt eine schematische Schnittansicht des Schlusses 16 gemäß Figur 9. Bei dem aufgelegten Schluss 16 ist vorgesehen, dass der Schlussabschnitt 23 der Branche 14 und der Schlussabschnitt 24 der Branche 15 seitlich aneinander vorbeigeführt werden. Die Schlussabschnitte 23, 24 können in der Breite zu den Griffabschnitten 18, 19, reduziert, beispielsweise halbiert, sein. Bei den Darstellungen in Figur 9 und 10 ist die Verriegelungseinrichtung 28 verriegelt. Das Verriegelungselement 31 an dem Verriegelungshebel 29 greift an dem Verriegelungselement 32 der Branche 15 bzw. an dem Schlussabschnitt 24 an. In dem Schlussabschnitt 23 ist eine Aussparung vorgesehen, innerhalb welcher der Verriegelungshebel 29 ausgehend von der Schwenkachse 36 bis zum Verriegelungselement 31 positioniert sein kann. Außerhalb des Griffabschnittes 18 ist ein Betätigungsabschnitt 34 des Verriegelungshebels 29 positioniert. Dieser liegt vorzugsweise an der Außenseite des Griffabschnitts 18 in Analogie zu der Ausführungsform in den Figuren 1 bis 4. Die Verriegelungseinrichtung 28 wirkt auf den Betätigungsabschnitt 34 des Verriegelungshebels 29 und hält die Verriegelungseinrichtung 28 in der Verriegelungsposition. Der Aufbau der Verriegelungseinrichtung 28 entspricht dem in den Figuren 5 und 6. Ein federgelagertes Druckstück 38 ist in dem Griffabschnitt 18 angeordnet.

Die Figur 11 zeigt eine schematische Schnittansicht des aufgelegten Schlusses 16, wobei die Verriegelungseinrichtung 28 durch Drücken des Betätigungsabschnittes in die Entriegelungsposition übergeführt ist. Die Verriegelungselemente 31, 32 sind außer Eingriff gebracht. Ausgehend von dieser Anordnung ist eine Schwenkbewegung der Branchen 14, 15 in eine weitere Arbeitsposition möglich, wie dies in Figur 1 dargestellt ist. Bei einer Nichtbetätigung des Verriegelungshebels 29 kehrt die Verriegelungseinrichtung 28 in die Verriegelungsposition zurück und verhindert ein Aufeinanderzuführen der Griffabschnitte 18, 19. Durch die Ausbildung der Verriegelungselemente 31, 32 ist es jedoch möglich, dass ohne Betätigung der Verriegelungseinrichtung 28 die Griffabschnitte 18, 19 weiter voneinander wegbewegt werden können.

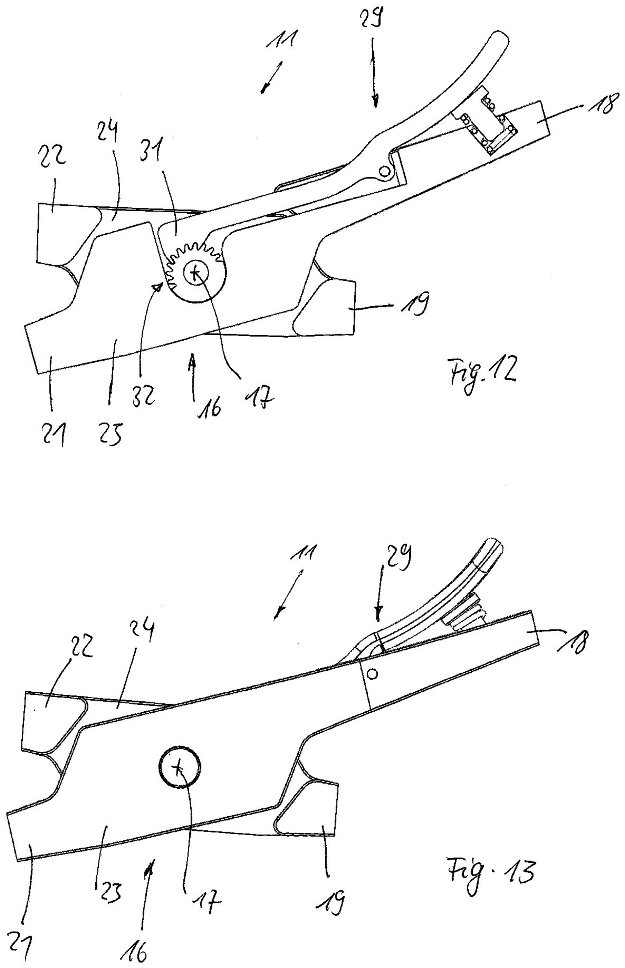

In Figur 13 ist eine schematische Seitenansicht der Schlussabschnitte 23, 24 der Anordnung gemäß Figur 12 von dem aufgelegten Schluss 16 dargestellt.

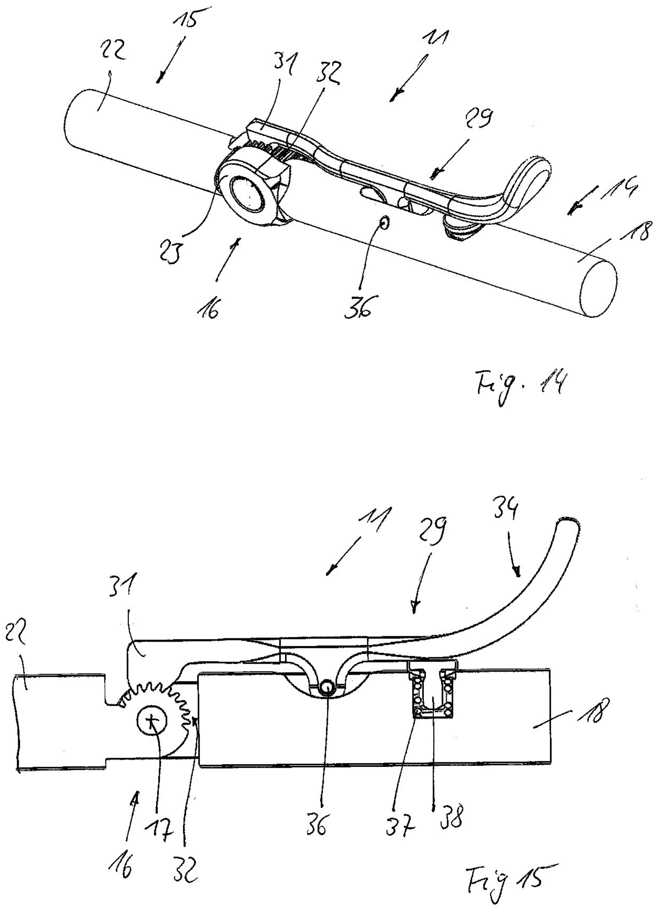

In den Figuren 14 bis 17 ist eine alternative Ausführungsform des aufgelegten Schlusses 16 zu den Figuren 9 bis 13 dargestellt. Diese Ausführungsform weicht in der konkreten Ausgestaltung der Schlussabschnitte 23, 24 als auch in der Positionierung des Verriegelungshebels 29 ab. Die Figur 14 zeigt eine perspektivische Ansicht des alternativen Schlusses 16. In Figur 15 ist eine schematische Schnittansicht des Schlusses 16 gemäß Figur 14 dargestellt. An dem Griffabschnitt 18 der Branche 14 ist der Verriegelungshebel 29 der Verriegelungseinrichtung 28 angeordnet. Gegenüberliegend ist ein Funktionsabschnitt 22 mit dem Schlussabschnitt 24 der Branche 15 dargestellt.

Bei dieser Ausführungsform ist vorgesehen, dass der Verriegelungshebel 29 nur in Bezug auf eine Schwenkachse 36 in dem Griffabschnitt 18 vorgesehen ist. Die weiteren Abschnitte des Verriegelungshebels 29 sind außerhalb des Griffabschnittes 18 und des Schlussabschnittes 23 angeordnet. Das Druckstück 38 und das dazu angeordnete Kraftspeicherelement 37 der Verriegelungseinrichtung 28 sind in Analogie zu der vorbeschriebenen Ausführungsform gemäß Figur 9 bis 13 angeordnet, wobei im Ausführungsbeispiel gemäß den Figuren 14 bis 17 die Bewegungsachse des Druckstückes 38 rechtwinklig zur Längsachse des Griffabschnittes 18 ausgerichtet ist.

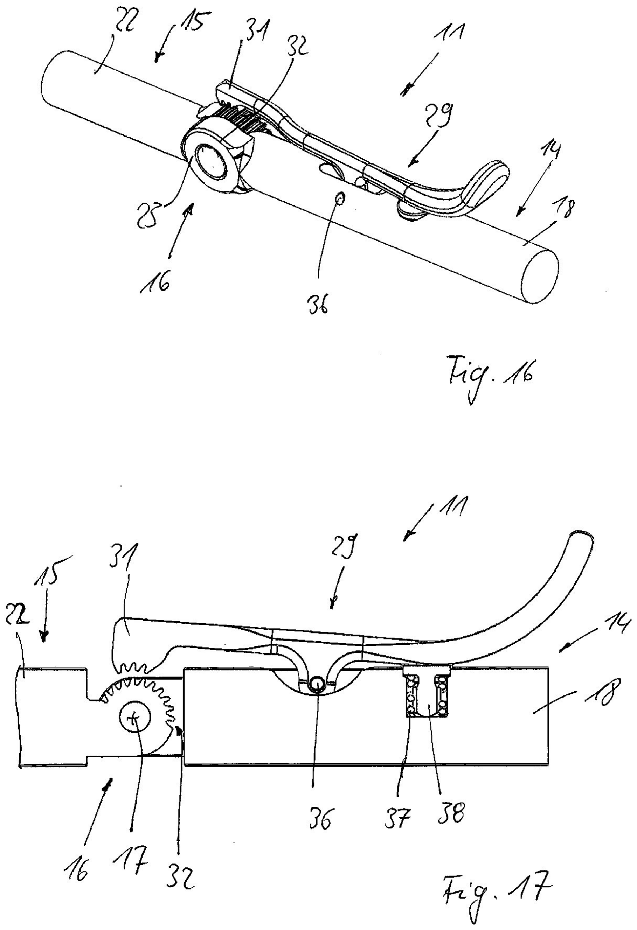

In Figur 16 ist die alternative Ausführungsform des Schlusses 16 gemäß Figur 14 mit der Verriegelungseinrichtung 28 in einer Entriegelungsposition dargestellt. Die Figur 17 zeigt eine schematische Schnittansicht zu Figur 16, um die Funktionsweise und die Anordnung der Verriegelungseinrichtung 28 in der Entriegelungsposition zu verdeutlichen.

Die Funktions- und Wirkungsweise der alternativen Ausführungsform des Schlusses 16 gemäß den Figuren 14 bis 17 entspricht den vorbeschriebenen Ausführungsformen.

Alle vorbeschriebenen Ausführungsformen haben gemeinsam, dass das eine Verriegelungselement 32 fest an dem Schlussabschnitt 24 angeordnet und zur Drehachse 17 ausgerichtet ist und das andere Verriegelungselement 31 an dem Verriegelungshebel 29 in einer Entriegelungsposition um die Kreisbahn des fest an dem Schlussabschnitt 2 angeordneten Verriegelungselementes 32 drehbar ist.

## Patentansprüche

1. Medizinisches Instrument,
- mit zwei Branchen (14, 15), die jeweils einen Griffabschnitt (18, 19) und einen Funktionsabschnitt (21, 22) aufweisen, zwischen denen ein Schlussabschnitt (23, 24) vorgesehen ist,
- mit einem Schluss (16), durch welchen die Branchen (14, 15) schwenkbar um eine gemeinsame Drehachse (17) verbunden sind, wobei die Schlussabschnitte (23, 24) mit einem Verbindungselement (26) schwenkbar in der Drehachse (17) gelagert sind,
- mit einer Verriegelungseinrichtung (28), welche zwei voneinander lösbare Verriegelungselemente (31, 32) aufweist, wobei eines der Verriegelungselemente (31) mit einem Verriegelungshebel (29) betätigbar ist,
**dadurch gekennzeichnet,**
- **dass** zumindest die Verriegelungselemente (31, 32) der Verriegelungseinrichtung (28) an oder in dem Schluss (16) angeordnet sind.

2. Medizinisches Instrument nach Anspruch 1, **dadurch gekennzeichnet, dass** zumindest die Verriegelungselemente (31, 32) der Verriegelungseinrichtung (28) innerhalb dem Schluss (16) integriert sind.

3. Medizinisches Instrument nach Anspruch 1 oder 2, **dadurch gekennzeichnet, dass** die Verriegelungselemente (31, 32) einander zuweisende Rastelemente (33) aufweisen, die ineinandergreifen.

4. Medizinisches Instrument nach Anspruch 3, **dadurch gekennzeichnet, dass** die Rastelemente (33) der Verriegelungselemente (31, 32) derart ausgebildet sind, dass eine Schwenkbewegung der Branchen (14, 15) in eine Richtung, insbesondere in eine Schließrichtung der Funktionsabschnitte (21, 22) gesperrt und eine Schwenkbewegung der Branchen (14, 15) in die entgegengesetzte Richtung, insbesondere in eine Aufspreizrichtung der Funktionsabschnitte (21, 22), freigegeben wird.

5. Medizinisches Instrument nach einem der vorhergehenden Ansprüche, **dadurch gekennzeichnet, dass** das eine Verriegelungselement (32) fest an dem einen Schlussabschnitt (24) ausgebildet ist, und das andere an dem Verriegelungshebel (29) angeordnete Verriegelungselement (31) lösbar dazu ist.

6. Medizinisches Instrument nach einem der vorhergehenden Ansprüche, **dadurch gekennzeichnet, dass** der Verriegelungshebel (29) an dem Griffabschnitt (18) schwenkbar gelagert ist, welcher dem Griffabschnitt (19) mit dem in dem Schlussabschnitt (24) fest angeordneten Verriegelungselement (32) gegenüberliegt.

7. Medizinisches Instrument nach einem der vorhergehenden Ansprüche, **dadurch gekennzeichnet, dass** der Verriegelungshebel (29) an einer Außenseite des Griffabschnitts (18) der Branche (14) sich erstreckt, sodass die Griffabschnitte (18, 19) in einer Arbeitsposition, insbesondere Aufspreizposition, aneinander anliegen.

8. Medizinisches Instrument nach einem der vorhergehenden Ansprüche, **dadurch gekennzeichnet, dass** der Verriegelungshebel (29) in einer Schwenkachse (36) parallel zur Drehachse (17) schwenkbar gelagert ist, wobei zumindest das daran angeordnete Verriegelungselement (31) innerhalb dem Schlussabschnitt (23) angeordnet ist.

9. Medizinisches Instrument nach einem der vorhergehenden Ansprüche, **dadurch gekennzeichnet, dass** der Verriegelungshebel (29) selbsthaltend durch ein Kraftspeicherelement (37) in einer Verriegelungsposition angeordnet ist, sodass die Verriegelungselemente (31, 32) ineinandergreifen oder aneinander anliegen.

10. Medizinisches Instrument nach Anspruch 9, **dadurch gekennzeichnet, dass** das Kraftspeicherelement (37) als ein Federelement ausgebildet ist.

11. Medizinisches Instrument nach Anspruch 9 oder 10, **dadurch gekennzeichnet, dass** in dem Schlussabschnitt (23), innerhalb welchem sich vorzugsweise zumindest teilweise der Verriegelungshebel (29) erstreckt, ein mit dem Kraftspeicherelement (37) gelagertes Druckstück (38) vorgesehen ist, welches an dem durch den Verriegelungshebel (29) betätigbaren Verriegelungselement (31) angreift oder anliegt.

12. Medizinisches Instrument nach einem der Ansprüche 9 bis 11, **dadurch gekennzeichnet, dass** das Kraftspeicherelement (37) und das damit beaufschlagte Druckstück (38) entlang einer Achse verschiebbar in dem Schlussabschnitt (23) gelagert sind, welche senkrecht zur Drehachse (17) ausgerichtet ist und insbesondere die Drehachse (17) schneidet.

13. Medizinisches Instrument nach einem der vorhergehenden an Ansprüche, **dadurch gekennzeichnet, dass** der Verriegelungshebel (29) sich bis zu einer Grifföffnung (30) des Griffabschnitts (18) erstreckt.

14. Medizinisches Instrument nach einem der vorhergehenden Ansprüche, **dadurch gekennzeichnet, dass** die Funktionsabschnitte (21, 22) der Branchen (14, 15) jeweils eine Schnittstelle (42) zur lösbaren Anordnung von Funktionselementen (43, 44) aufweist.

15. Medizinisches Instrument nach einem der vorhergehenden Ansprüche, **dadurch gekennzeichnet, dass** die Schlussabschnitte (23, 24) des Schlusses (16) als ein aufgelegter Schluss, ein Rollenschluss oder als ein Durchsteck- oder Kastenschluss ausgebildet sind.
